Europäisches Patentamt

European Patent Office

Office européen des brevets

(19)

(11) Veröffentlichungsnummer: **0 052 322 B1**

# EUROPÄISCHE PATENTSCHRIFT

(45) Veröffentlichungstag der Patentschrift:
27.03.85

(51) Int. Cl.⁴: **A 61 K 9/50**

(21) Anmeldenummer: **81109575.1**

(22) Anmeldetag: **09.11.81**

(54) Verfahren zur Herstellung von Lipid-Vesikeln durch Ultraschallbehandlung, Anwendung des Verfahrens und Vorrichtung zur Durchführung des Verfahrens.

(30) Priorität: **10.11.80 DE 3042360**

(43) Veröffentlichungstag der Anmeldung:
**26.05.82 Patentblatt 82/21**

(45) Bekanntmachung des Hinweises auf die Patenterteilung:
**27.03.85 Patentblatt 85/13**

(84) Benannte Vertragsstaaten:
**AT BE CH DE FR GB LI NL SE**

(56) Entgegenhaltungen:
**DE - A - 2 338 503**

**FOLIA BIOCH. ET BIOL. GRAECA, Special Issue, Band XIV, 1978**

(73) Patentinhaber: **Gersonde, Klaus, Prof. Dr., Preusweg 69, D-5100 Aachen (DE)**

(72) Erfinder: **Gersonde, Klaus, Prof. Dr. med., Preusweg 69, D-5100 Aachen (DE)**
Erfinder: **Schäl, Wilfried, Dr., Tannenwaldweg 27, D-6380 Bad Homburg (DE)**

(74) Vertreter: **Biermann, Wilhelm, Dr.-Ing., Morillenhang 39, D-5100 Aachen (DE)**

## Beschreibung

Die Erfindung betrifft ein Verfahren zur Herstellung von uniformen, unilamellaren sogenannten kleinen Lipid-Vesikeln, insbesondere ein Verfahren zum Überführen von lamellär angeordneten Lipiden in Lipidvesikel, bei dem die zu desintegrierenden Lipid-Strukturen (Lamellen) in einer Suspensionsflüssigkeit innerhalb eines Beschallungsgefäßes bei im wesentlichen konstanter Temperatur einer Ultraschallbehandlung unterworfen werden. Die Erfindung umfaßt ferner geeignete Vorrichtungen zur Durchführung des Verfahrens.

Lipid-Vesikel werden zum Beispiel für medizinisch-therapeutische und wissenschaftliche Zwecke benötigt. Arzneimittel, die einen intrazellulären Wirkort haben, müssen die Zell-Membran passieren können. Viele Effektoren, die den intrazellulären Stoffwechsel kontrollieren und in der Zelle gebildet werden, können die Zelle weder verlassen noch in diese von außen eindringen. Der therapeutische Einsatz dieser Stoff-Klasse macht daher einen Transportmechanismus erforderlich, der es erlaubt, nichtmembran-permeable Stoffe in die Zellen hineinzuschleusen, ohne daß diese Stoffe die Zellen wieder verlassen können. Ein solcher Transportmechanismus soll darüber hinaus möglichst unabhängig von dem zu transportierenden Wirkstoff sein, also jede Art von Effektor transportieren können, andererseits aber Zell-spezifisch sein, d. h. den Transport nur in bestimmte Zellen ermöglichen. Ein Transport-System mit den obengenannten Eigenschaften stellen Lipid-Vesikel dar, welche die verschiedensten Stoffe einschließen können, wie Enzyme, Arzneimittel, Chelat-bildende Substanzen, Hormone, Zell-Effektoren, Antigene, Antikörper, Interferon-Induktoren und Gene. In den Lipid-Vesikeln sind das Lösungsmittel und die im Lösungsmittel gelösten Stoffe von Phospholipid-Doppelschichtmembranen umschlossen. Die Lipid-Membran hat eine Dicke von 4 nm, die Vesikel können einen Durchmesser von 25 bis 120 nm annehmen. Die Größe der Vesikel läßt sich mit Hilfe der Laser-Lichtstreuung, durch Ultrazentrifugation, Gelfiltration oder im Raster-Elektronenmikroskop bestimmen.

Ein wichtiges Anwendungsgebiet der Lipid-Vesikel ist die Inkorporierung von Inositolhexaphosphat (IHP) in rote Blutzellen nach dem von Y. C. Nicolau und K. Gersonde beschriebenen Verfahren (US-Patent 4 192 869), zur Herabsetzung der Sauerstoff-Affinität des Hämoglobins. Man weiß nämlich, daß z. B. bei der Lagerung von Blutkonserven die Sauerstoff-Affinität des Hämoglobins in den roten Blutzellen ständig zunimmt. Ebenso beobachtet man bei bestimmten Krankheiten eine erhöhte Sauerstoff-Affinität des Hämoglobins. Diese erhöhte Sauerstoff-Affinität führt dazu, daß nur ein geringer Anteil des Sauerstoffs, der an Hämoglobin gebunden ist und im Blut zirkuliert, tatsächlich an das Gewebe abgegeben wird. Diese hohe $O_2$-Affinität

des Hämoglobins kann durch Bindung von bestimmten Effektoren an das Hämoglobin herabgesetzt werden. Der stärkste Effektor dieser Art ist das Inositolhexaphosphat (IHP). Die Inkorporierung von IHP wird erreicht dadurch, daß intakte Zellen mit IHP-beladenen Lipid-Vesikeln inkubiert werden, wobei durch Fusion der Lipid-Membranen der Zelle und der Vesikel IHP in die Zelle eingeschleust wird und dort seine Wirkung erzielt, nämlich die Veränderung der $O_2$-Affinität des Hämoglobins, meßbar als »Rechtsverschiebung« der Hämoglobin-$O_2$-Dissoziationskurve. Nach Rückkehr dieser IHP-beladenen roten Blutzellen in den Kreislauf wird ein erheblich höherer Anteil der in den roten Blutzellen gespeicherten $O_2$-Menge in der Peripherie abgegeben. Diese Eigenschaft der modifizierten roten Blutzellen bleibt während des gesamten Lebens der Zelle erhalten.

Für die Inkorporierung von Inositolhexaphosphat in rote Blutzellen werden kleine, unilamellare IHP-beladene Lipid-Vesikel mit einem Durchmesser von 20 bis 50 nm benötigt. Es ist bekannt, Lipid-Vesikel durch Desintegration von Lipid-Suspensionen im Ultraschallfeld herzustellen. Der Fortschritt in der Anwendung von Lipid-Vesikeln in der Therapie vollzieht sich bisher nur sehr langsam, da die Herstellung von für die Fusion mit den roten Blutzellen geeigneten Lipid-Vesikeln in ausreichender Menge mit erheblichen Schwierigkeiten verbunden ist. Die für diesen Zweck geeigneten Lipid-Vesikel müssen nämlich nicht nur in großen Mengen hergestellt werden können, sondern auch reproduzierbar von einheitlicher Größe und somit dosierbar in der therapeutischen Anwendung sein. Die nachträgliche Anwendung von Trennverfahren zum Abtrennen geeigneter Fraktionen der Lipid-Vesikel wirft vielerlei Probleme auf, z. B. Aufrechterhaltung der Sterilität und Anwendung aufwendiger und zeitraubender Trenn-Techniken, die die biologische Effektivität der Vesikel, die nur eine Halb-Lebenszeit von ca. 1 Tag haben, stark vermindern. Das einzige Verfahren, das die Herstellung großer Mengen von Vesikeln in kurzer Zeit erlaubt, ist die Desintegration im Ultraschall. Außer von der Art und Zusammensetzung der Lipid-Membran der Vesikel hängen der Erfolg und die Reproduzierbarkeit der wissenschaftlichen Untersuchung bzw. der therapeutischen Behandlung, d. h. Einschleusung von IHP in rote Blutzellen, wesentlich von der Größe der Lipid-Vesikel ab. Die Kontrolle, ob sich bei der Desintegration der Lipid-Suspension die Lipid-Vesikel in ausreichender Homogenität und somit Qualität und in ausreichender Menge gebildet haben, erfolgt in der Weise, daß man mit den hergestellten Lipid-Vesikeln die gewünschten IHP-Einschleusungsversuche in rote Blutzellen durchführt, das intrazelluläre IHP chemisch nachweist und die Hämoglobin-$O_2$-Dissoziationskurve intakter Zellen mißt bzw. die gewünschte biologische oder therapeutische Wirkung der IHP-beladenen roten Blutzellen im Tierversuch nachweist. Die Ergebnisse der Versuche bzw. der Erfolg der Behandlung können also erst nach aufwendigen Experimenten beurteilt werden, und der Erfolg der Ultraschallbehandlung, nämlich die Herstellung von für die Fusion mit den roten Blutzellen befähigten Vesikeln, erst im Nachhinein erkannt werden.

Es hat sich gezeigt, daß die Herstellung von Lipid-Vesikeln — insbesondere in großen Volumina, wie sie für therapeutische Verfahren erforderlich sind — mit gleichbleibenden Eigenschaften mit Hilfe der bekannten Ultraschall-Technik schwierig ist. Trotz Einhaltung anscheinend völlig gleicher äußerer Bedingungen bei der Desintegration der Lipid-Suspensionen im Ultraschallfeld gelingt es bisher nicht, stets die gleiche Ausbeute an sogenannten kleinen unilamellaren und somit fusionswirksamen Lipid-Vesikeln zu erhalten.

Der Erfindung liegt die Aufgabe zugrunde, das eingangs genannte Verfahren zur Herstellung von Lipid-Vesikeln, die erfolgreich Effektoren in Zellen einschleusen, dahingehend weiterzuentwickeln, daß der Wirkungsgrad des Verfahrens erhöht wird, und daß in reproduzierbarer Weise eine hohe Ausbeute an für den jeweils gewünschten Zweck hochwirksamen Lipid-Vesikeln erzielt wird.

Die Erfindung besteht darin, daß die für den gewünschten Zweck wirksamste Vesikelgröße bzw. Vesikelgrößenverteilung, sowie die für die Erzielung dieser Vesikelgröße bzw. Vesikelgrößenverteilung optimale Ultraschall-Frequenz und -Intensität ermittelt wird, und daß die so ermittelte optimale Ultraschall-Frequenz und -Intensität der bei im übrigen gleichen Behandlungsbedingungen dadurch konstantgehalten wird, daß während der Ultraschallbehandlung der Istwert der Frequenz und der Intensität des Ultraschallfeldes in dem Reaktionsmedium fortlaufend gemessen, und in Abhängigkeit von diesem Istwert die Ausgangsleistung und die Frequenz des den Schwingungsgeber speisenden elektrischen Generators geregelt wird.

Der Erfindung liegt die Erkenntnis zugrunde, daß bei Verwendung der Vesikel zu therapeutischen Zwecken Größe bzw. Größenverteilung der Vesikel für die Wirksamkeit derselben von ausschlaggebender Bedeutung ist, und daß andererseits die Größe und Homogenität der im Ultraschallfeld erzeugten Vesikel in empfindlicher Weise von der Konstanz und Intensität des auf die Lipide einwirkenden Ultraschallfeldes abhängen. Während es bisher üblich ist, die in den Schallgeber eingekoppelte Schallenergie zu messen und konstant zu halten, schlägt die Erfindung vor, die in dem Reaktionsmedium wirksame Schallenergie zu messen und gezielt zu verändern, und zwar in der Weise, daß die effektive Schallenergie im Reaktionsmedium selbst auf ihrem optimalen Wert konstantgehalten wird. Durch das Messen der effektiven Schallenergie im Reaktionsmedium selbst, und durch die Verwendung dieses Istwertes zum Regeln der eingekoppelten Schallenergie, werden alle Einflüsse kompensiert, die die effektive Schallintensität und die Schallfrequenz am Vesikel-Bildungsort beeinflussen, wie z. B. unterschiedliche und sich verhindernde Absorption der Schallenergie mit fortschreitender Reaktion, sich ändernde geometrische Verhältnisse innerhalb des Reaktionsmediums bei Auftreten von Gasbläschen während der Ultraschallbehandlung, sowie Reflexion eines Frequenzbandes von Schallwellen, das sich mit den vom Schallgeber abgestrahlten Schallwellen überlagert, und gegebenenfalls bei phasenverschobener Überlagerung bis zur Auslöschung der Schallenergie führt.

Wenn man also gemäß dem erfindungsgemäßen Verfahren zunächst mit Hilfe der bekannten Untersuchungsmethoden den für den jeweiligen Zweck optimalen Vesikeldurchmesser bzw. die optimale Verteilungskurve der wirksamsten Vesikeldurchmesser feststellt, sodann in einer weiteren Versuchsreihe die optimalen Ultraschallbedingungen wie Frequenz und Schalldruck ermittelt, die zu der gewünschten Verteilungskurve führen, und dann die so ermittelte optimale Ultraschallintensität und Ultraschallfrequenz innerhalb des Reaktionsmediums während der Reaktionsdauer konstant hält, erhält man eine sehr hohe Ausbeute an für den jeweiligen Zweck außerordentlich wirkungsvollen Lipid-Vesikeln.

Das erfindungsgemäße Verfahren läßt sich nicht nur für die Herstellung von Lipid-Vesikeln, sondern mit demselben Erfolg auch zum Behandeln von natürlichen biologischen Membranen und deren Umwandlung in Vesikel anwenden, beispielsweise bei der Erforschung von Funktion und Struktur von Membran-Enzymen. Auch hierfür ist es nämlich erforderlich, die Membran-Enzyme reproduzierbar in Vesikel bestimmter Größe zu überführen, was nach den bekannten Verfahren nicht mit ausreichender Sicherheit möglich ist.

Weitere Einzelheiten und Vorteile des erfindungsgemäßen Verfahrens werden nachfolgend anhand der Zeichnungen und anhand von Ausführungsbeispielen näher beschrieben. In den Zeichnungen zeigt

Fig. 1 eine für die Herstellung von kleineren Mengen von Lipid-Vesikel-Suspensionen geeignete Vorrichtung mit den Merkmalen der Erfindung, teilweise in schematischer Darstellung, und

Fig. 2 eine für die Herstellung von Lipid-Vesikel-Suspensionen in Steril-Packungen für den klinischen Gebrauch in Liter-Quantitäten geeignete Vorrichtung, ebenfalls in teilweise schematischer Darstellung.

Das Reaktionsmedium 1 in Form einer Suspension eines Lipides in einem geeigneten Dispersionsmittel befindet sich in dem inneren zylinderförmigen Rohr 2 des doppelwandigen gläsernen Reaktionsgefäßes 3. Der Boden des Reaktionsgefäßes 3 weist eine zu dem Reaktionsraum 3 durchgehende Öffnung auf, in die ein Schallaufnehmer 5 eingesetzt ist. Der Schallaufnehmer 5 ist mit Hilfe einer Epoxidharzschicht 6 mit der die Öffnung umgebenden Wand 7 verklebt und abgedichtet. Von oben taucht in das Reaktionsmedium 1 ein Ultraschall-Schwingungsgeber 8 ein. Der aus dem Reaktionsgefäß 3 oben herausragende Teil 9 des Schwingungsgebers 8 ist mit einer Kupplung 9 für den Stromanschluß versehen.

Durch die doppelte Wand des Reaktionsgefäßes wird ein Hohlraum 10 gebildet, der von Kühlwasser durchströmt ist. Die Rohrstutzen 11 und 12 dienen zur Zuleitung bzw. zur Abführung des Kühlwassers. Der Rohrstutzen 13 führt in den Reaktionsraum oberhalb des Reaktionsmediums 1 und dient zur Zuführung eines Inertgases wie Argon. Nach oben ist der Reaktionsraum abgeschlossen durch einen Deckel 14. Durch die zwischen dem Deckel 14 und dem Ultraschallgeber bzw. der Gefäßwand verbleibenden Spalte kann das unter geringem Überdruck stehende Inertgas entweichen.

Der Schallaufnehmer 5 weist als eigentlichen Schalldruck- bzw. Schallintensitätsempfänger an seinem oberen, mit dem Reaktionsmedium 1 in Kontakt stehenden Ende eine Piezo-Scheibe 18 auf, die in dem dargestellten Fall radial in einem Halterohr eingebaut, und in diesem Halterohr beispielsweise mit Epoxidharz eingeklebt und abgedichtet ist. Das von der Piezo-Scheibe 18 gelieferte elektrische Signal ist ein Maß für die effektive Schallfrequenz und die effektive Schallintensität in dem Reaktionsmedium. Dieses elektrische Signal stellt den Istwert des Regelkreises dar und wird über die Leitung 20 dem Regelverstärker 21 zugeführt. Auf dem Oszillographen 22 können gegebenenfalls die effektive Frequenz und die Schallintensität visuell verfolgt, und erforderlichenfalls von Hand Nachregulierungen der Frequenz und der Ausgangsleistung des Hochfrequenzgenerators 23 vorgenommen werden.

Dem Regelverstärker 21 wird über eine Sollwert-Einstell-Vorrichtung 24 der Sollwert für die Ultraschall-Intensität, und über eine Sollwert-Einstell-Vorrichtung 25 der Sollwert für die Ultraschall-Frequenz vorgegeben. Bei Abweichungen der Istwerte der Frequenz und der Intensität des Ultraschallfeldes im Reaktionsmedium von den vorgegebenen Sollwerten wird über die Leitung 26 der Ultraschallgenerator 23 angesteuert, dessen Frequenz und/oder Ausgangsleistung so lange verändert werden, bis die innerhalb des Reaktionsmediums 1 gemessenen effektiven Werte mit den vorgegebenen Sollwerten übereinstimmen. Von dem Hochfrequenzgenerator 23 wird der Ultraschallgeber 8 über die Leitung 28 mit der erforderlichen elektrischen Spannung versorgt.

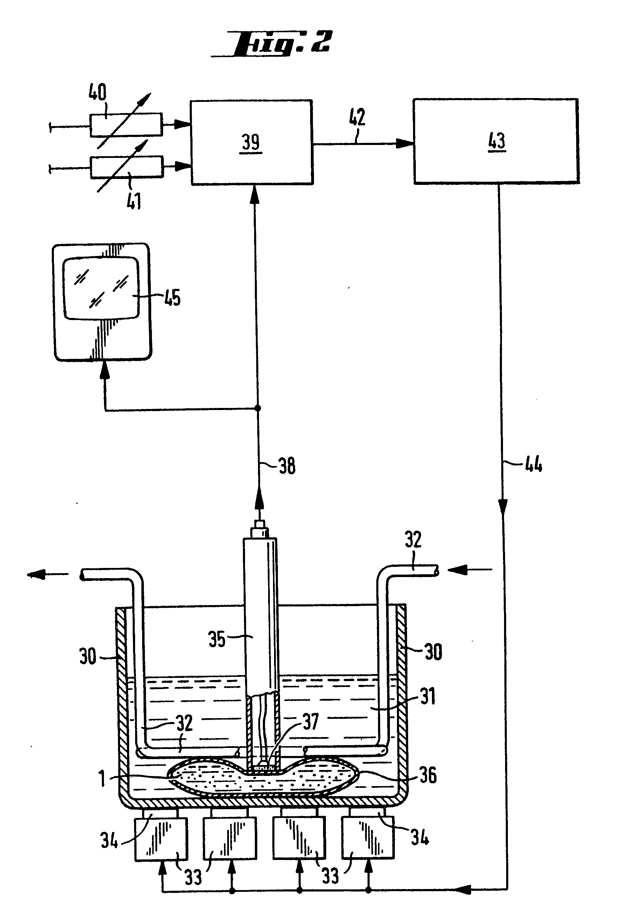

Die in Fig. 2 dargestellte Vorrichtung eignet sich für die Herstellung von Lipid-Vesikeln in größeren Quantitäten. Das Reaktionsgefäß ist eine oben offene zylindrische rechteckige Wanne 30 aus korrosionsbeständigem Cr-Ni-Stahl. Die Kühlung der Flüssigkeit 31 in dem Reaktionsgefäß erfolgt durch eine Kühlschlangenanordnung 32, die in die in der Wanne 30 befindliche Flüssigkeit 31 eingesetzt wird. Unter dem Boden der Wanne 30 sind auf der Außenseite acht elektro-akustische Wandler 33 angeordnet, deren Schwingungsgeber 34 an den Boden der Wanne 30 angekoppelt sind. Die Schallenergie wird so auf die Flüssigkeit 31 in der Wanne 30 übertragen. In die Flüssigkeit 31 taucht von oben der Schallaufnehmer 35 ein. Das Reaktionsmedium selbst befindet sich in einem verschlossenen Steril-Beutel 36, der durch die Kühlschlangenanordnung 32 innerhalb der Flüssigkeit 31 wenig oberhalb des Bodens der Wanne 30 gehalten wird. Der Schallaufnehmer 35 wird so weit abgesenkt, daß der Schallaufnahmekopf, d. h. die Piezo-Scheibe 37, gegen den Steril-Beutel 36 gedrückt wird, und so das Ultraschallfeld in dem Reaktionsmedium erfaßt.

Das von der Piezo-Scheibe 37 gelieferte Signal steuert über die Leitung 38 den Regelverstärker 39 an und liefert diesem die Istwerte für den Regelvorgang. Die Sollwerte für die Frequenz und die Intensität des Ultraschalls werden vorgegeben durch die Sollwert-Vorgabeeinrichtung 40 bzw. 41. Bei Abweichung der Istwerte von den vorgegebenen Sollwerten wird über die Leitung 42 der regelbare elektrische Generator 43 angesteuert, dessen an die elektromagnetischen Wandler 33 über die Leitung 44 abgegebene Ausgangsleistung und/oder Frequenz so lange verändert werden, bis Sollwerte und Istwerte übereinstimmen. Die von der Piezo-Scheibe 37 gemessene Frequenz und Amplitude des Ultraschallfeldes können auf dem Oszillographen 45 visuell beobachtet werden, so daß auch ggf. ein manueller Eingriff in die Regelung möglich ist.

Mit Hilfe der beschriebenen Vorrichtungen werden beispielsweise folgende Reaktionen durchgeführt:

### Beispiel 1

Es sollen IHP-beladene Lipid-Vesikel für therapeutische Zwecke hergestellt werden, und zwar zur Einschleusung von IHP in rote Blutzellen mittels Fusion zur Verbesserung der $O_2$-Freisetzungseigenschaften der roten Blutzellen. Die generelle Methode zur Präparation von Lipid-Vesikeln ist in der US-Patentschrift 4 192 869 beschrieben, auf die insoweit Bezug genommen wird. Die Lipid-Vesikel sind aus Phosphatidylcholin, Phosphatidylserin und Cholesterol in molaren Verhältnissen von 8 : 2 : 7 aufgebaut. Diese Lipide werden zunächst in einem organischen Lösungsmittel aus 95 Teilen Chloroform und 5 Teilen Methanol gelöst, um eine homogene Lösung und Mischung dieser Lipide zu erreichen. Dann wird das Lösungsmittel bei 20 Grad Celsius im Rotationsverdampfer entfernt. Der dann im Rundkolben verbleibende Lipid-Film wird mit einer wäßrigen Lösung, welche die biologisch aktive Substanz (hier IHP) enthält, aufgenommen und geschüttelt, so daß sich nunmehr ebene Lipid-Lamellen in dieser Suspension bilden. Diese Suspension enthält Lipide in einer Konzentration von ca. 17–200 µg/l. Die Suspension ist ferner gesättigt an IHP und zwischen pH 7.0–8.0 gepuffert.

In einer vorausgehenden Versuchsreihe wurde festgestellt, daß sich für die Fusion mit Erythrozyten und die Inkorporierung von IHP in Erythrozyten Vesikel eignen, die die oben beschriebene Zusammensetzung haben und die einen Durchmesser von 25–50 nm (250–500 Å) aufweisen. Die Wirksamkeit ist um so größer, je größer der Mengenanteil dieser Vesikel-Formation in dem jeweiligen Präparat ist. Durch eine weitere Versuchsreihe wurde sodann ermittelt, daß sich diese gewünschte Durchmesserverteilung erreichen läßt, wenn die Lipide bei einer in etwa konstanten Temperatur von 37 Grad Celsius mit einer schmalbandigen Schallfrequenz von 20 kHz mit einer effektiven Schallenergie von 3 bis 6 W/cm² beschallt werden.

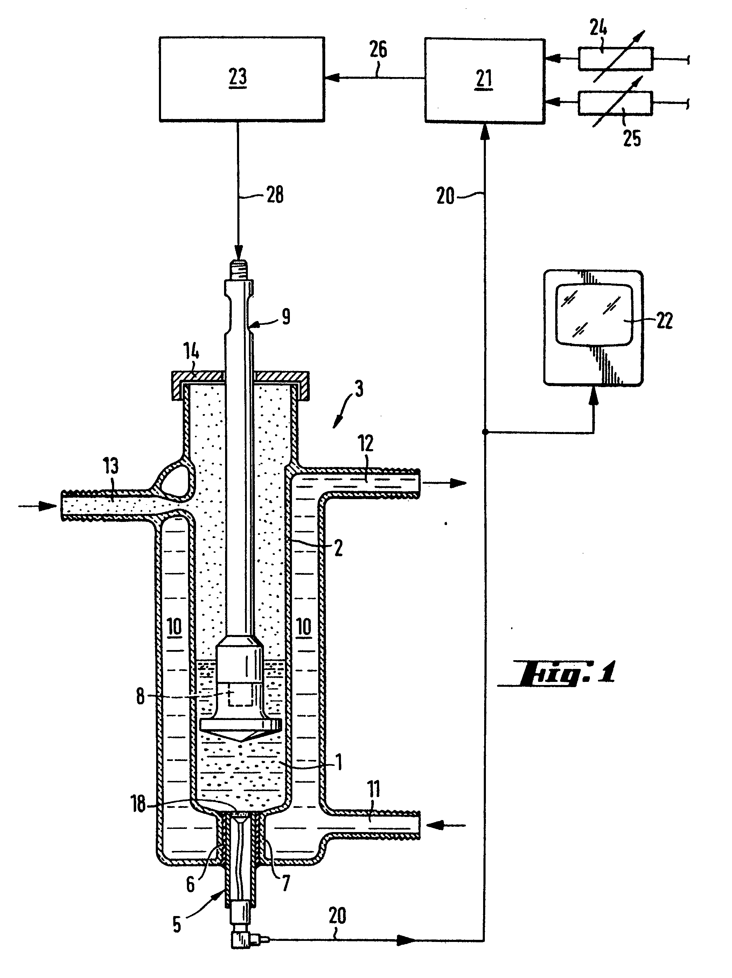

Die oben beschriebene Lipid-Suspension wird unter Inertgas in das Reaktionsgefäß 3 (Fig. 1) eingefüllt. Das Reaktionsgefäß wird durch das Rohr 13 mit Argon gespült. An den Sollwert-Einstellvorrichtungen 24 und 25 werden sodann die optimale Schallintensität und die gewünschte Schallfrequenz eingestellt. Die beschriebene Regeleinrichtung sorgt dafür, daß die optimale Schallintensität als effektive, auf die Reaktionsflüssigkeit zur vollen Einwirkung kommende Schallintensität während der gesamten Behandlungszeit eingehalten wird. Die Behandlung dauert 30 bis 60 Minuten. Während dieser Zeit wird durch das Kühlwasser die in dem Reaktionsgefäß entwickelte Wärme abgeführt und so die Temperatur konstant gehalten.

Die auf diese Weise erhaltene Vesikel-Suspension wird nun mit roten Blutzellen bei 37 Grad Celsius 1 h inkubiert. Danach werden die roten Blutzellen in isotonischen Puffer pH 7,4 gewaschen und die Hämoglobin-$O_2$-Dissoziationskurve der modifizierten intakten Zellen gemessen. Der Erfolg der IHP-Inkorporierung wird als »Rechtsverschiebung« der Hämoglobin-$O_2$-Dissoziationskurve erkannt. Der durch IHP-Bindung an Hämoglobin maximal erreichbare $O_2$-Halbsättigungsdruck beträgt bei 37 Grad Celsius und pH 7.4 23,5 mbar (95 mmHg).

Der Erfolg der kontrollierten Ultraschall-Methode liegt vor allem darin, daß der maximale IHP-Inkorporierungseffekt mit Volumen-Verhältnissen RBC (Rote-Blutzellen): Vesikel oder mit Lipid-Konzentrationen erreicht wird, die nur noch 10% der Werte betragen, die bei der bisher üblichen unkontrollierten Ultraschallanwendung für eine erfolgreiche IHP-Inkorporierung eingesetzt werden mußten. Daraus resultiert ein erheblicher wirtschaftlicher Vorteil, da Lipide teuer sind und nicht wiederverwendet werden können. Darüber hinaus sind mit der bisher üblichen Methode keine reproduzierbaren Ergebnisse zu erzielen, die unbedingte Voraussetzung für eine sichere Dosierung in der Therapie sind.

## Beispiel 2

Bei Lipid-Vesikeln für therapeutische Zwecke besteht die Forderung nach absoluter Keimfreiheit. Da eine Sterilisation der fertigen Vesikel-Suspension auf äußerste Schwierigkeiten stößt, wird folgendes Verfahren angewandt: Die Ausgangskomponenten entsprechend dem Beispiel 1 werden zunächst sterilisiert, und unter sterilen Kautelen in einen ebenfalls sterilisierten Beutel aus Polyäthylen oder Weich-PVC-Folie von etwa 0,5 mm Wandstärke gefüllt. Der Beutel wird steril dicht verschlossen und dann in dem Ultraschallgefäß nach Fig. 2 der Ultraschallwirkung ausgesetzt, wobei der Raum zwischen den Gefäßwänden und dem Beutel mit Wasser ausgefüllt ist. Die Messung der Schallintensität erfolgt in diesem Fall an der Außenseite des Beutels. Unter sonst gleichen Bedingungen wie in Beispiel 1 beschrieben werden die gleichen Ergebnisse erzielt, wenn die den Schwingungsgebern zugeführte Leitung etwa um den Faktor 1,65 erhöht wird. Dieser bei der vorgegebenen Versuchsanordnung anhand der Messungen experimentell ermittelte Faktor repräsentiert die durch die Einbringung des Beutels eintretenden Leistungsverluste.

## Patentansprüche

1. Verfahren zur Herstellung von Lipid-Vesikeln aus biologischen Membranen oder Lipid-Suspensionen, bei dem die zu desintegrierenden Lipid-Suspensionen oder Lipid-Partikel in einer Dispersionsflüssigkeit innerhalb eines Behandlungsgefäßes bei im wesentlichen konstanter Temperatur einer Ultraschallbehandlung unterworfen werden, dadurch gekennzeichnet, daß die für den gewünschten Zweck wirksame Vesikelgröße bzw. Vesikelgrößenverteilung, sowie die für die Erzielung dieser Vesikelgröße bzw. dieser Vesikelgrößenverteilung optimale Ultraschall-Frequenz und -Intensität in der Dispersionsflüssigkeit ermittelt, und daß die so ermittelte optimale Ultraschall-Frequenz und -Intensität bei im übrigen gleichen Behandlungsbedingungen dadurch konstant gehalten wird, daß während der Ultraschallbehandlung der Istwert der Frequenz und Intensität des Ultraschallfeldes in dem Reaktionsmedium fortlaufend gemessen, und in Abhängigkeit von dem Istwert die Ausgangsleistung und die Frequenz des den Schwingungsgeber speisenden elektrischen Generators geregelt wird.

2. Verfahren nach Anspruch 1, dadurch gekennzeichnet, daß die Regelung des den Ultraschallgeber speisenden Generators mit Hilfe eines automatischen Regelkreises mit Sollwert-Istwert-Vergleich vorgenommen wird.

3. Verfahren nach Anspruch 1 oder 2, dadurch gekennzeichnet, daß zur Messung der Ultraschall-Intensität und -Frequenz ein in die Reaktionsflüssigkeit eintauchender piezoelektrischer Schallaufnehmer verwendet wird.

4. Verfahren nach Anspruch 3, dadurch gekennzeichnet, daß der piezoelektrische Schallaufnehmer am Boden des Reaktionsgefäßes so angeordnet wird, daß die Richtung seiner höchsten Empfindlichkeit gegen den Ultraschallgeber gerichtet ist, während der Ultraschallgeber in das obere Drittel des Mediums eintaucht.

5. Verfahren nach einem oder mehreren der Ansprüche 1 bis 4, dadurch gekennzeichnet, daß das Reaktionsmedium unter einer Inertgasatmosphäre behandelt wird.

6. Verfahren nach einem oder mehreren der Ansprüche 1 bis 5, dadurch gekennzeichnet, daß das Reaktionsmedium sterilisiert und in einem Steril-Beutel eingeschlossen, und der das Reaktionsmedium enthaltende Steril-Beutel innerhalb einer dem Ultraschallfeld ausgesetzten Flüssigkeit behandelt wird.

7. Anwendung des Verfahrens nach einem oder mehreren der Ansprüche 1 bis 6 zur Herstellung von für therapeutische Zwecke bestimmten, mit einem Wirkstoff wie Inositolhexaphosphat beladenen, zur Fusion mit roten Blutzellen befähigten Lipid-Vesikeln.

8. Vorrichtung zur Durchführung des Verfahrens nach Anspruch 1, mit einem die Reaktionsflüssigkeit aufnehmenden Behandlungsgefäß und mindestens einem von einem elektrischen Generator gespeisten elektroakustischen Wandler zur Erzeugung eines Ultraschallfeldes in dem Reaktionsmedium, dadurch gekennzeichnet, daß innerhalb des Behandlungsgefäßes (3; 30) als Schallaufnehmer ein akustisch-elektrischer Wandler (18; 37) angeordnet ist, dessen Ausgangssignal zur Regelung der Ultraschall-Intensität und -Frequenz in der Reaktionsflüssigkeit dient.

9. Vorrichtung nach Anspruch 8, gekennzeichnet durch einen den elektrischen Generator (23; 43) ansteuernden Regelverstärker (21; 39) mit Sollwert-Istwert-Vergleich.

10. Vorrichtung nach Anspruch 8 und 9, dadurch gekennzeichnet, daß der akustisch-elektrische Wandler (18; 37) aus einer am Ende eines Rohres angeordneten Piezo-Scheibe besteht, die mit ihrer gegen Schalldruck empfindlichsten Richtung gegen den Schwingungsgeber gerichtet ist.

11. Vorrichtung nach Anspruch 8 bis 10, dadurch gekennzeichnet, daß die Geometrie des Ultraschallgebers und des das Medium aufnehmenden Behandlungsgefäßes (3; 30) so gewählt sind, daß die durch Reflexion verursachte Bildung von Ultraschallwellen mit einer von der Sollfrequenz abweichenden Frequenz und/oder mit gegenüber den primären Ultraschallwellen verschobener Phase in dem beschallten Medium vermieden wird.

12. Vorrichtung nach Anspruch 8 bis 11, dadurch gekennzeichnet, daß das Behandlungsgefäß (3; 30) von einer Kühlflüssigkeit durchströmte Doppelwände aufweist.

13. Vorrichtung nach Anspruch 8 bis 12, dadurch gekennzeichnet, daß das Behandlungsgefäß (3) gegen die Außenatmosphäre abgeschlossen ist und eine Zuleitung (13) für die Zufuhr von Inertgas zu dem Gasraum oberhalb des beschallten Reaktionsmediums (1) aufweist.

## Claims

1. A method of preparing lipid vesicles from biological membranes or lipid suspensions, in which the lipid suspensions or lipid particles to be desintegrated are ultrasonically treated in a dispersion fluid inside a treatment container at a substantially constant temperature, characterized in that the size respectively size distribution of the lipid vesicles, effective for the desired purpose, and the optimum ultrasonic frequency and intensity in the dispersion fluid required for obtaining this size respectively this size distribution are determined, and that the thus determined optimum ultrasonic frequency and intensity, with the other constant conditions, are maintained constant in such a manner that during the ultrasonic treatment the actuel value of frequency and intensity of the ultrasonic field in the reaction medium is continuously measured and the output power and the frequency of the electric generator supplying the sound transmitter are controlled in dependence upon the actuel value of the frequency and intensity of the ultrasonic field.

2. A method as defined in claim 1, characterized in that control of the generator supplying the ultrasonic transmitter is realised by means of an automatic control circuit including an actuel value — nominal value comparator.

3. A method as defined in claim 1 or 2, characterized in that a piezoelectric sound receiver dipped in the reaction medium is used for measuring the ultrasonic intensity and frequency.

4. A method as defined in claim 3, characterized in that the piezoelectric sound receiver is arranged on the bottom of the reaction container so that its direction of the maximum sensivity is directed against the ultasound transmitter, the ultrasound transmitter being dipped into the upper third of the reaction medium.

5. A method as defined in a or more ot he claims 1 to 4, characterized in that the reaction medium is treated under on inert gas atmosphere.

6. A method as defined in on or more of the claims 1 to 5, characterized in that the reaction medium is sterilised and enclosed in a sterile bag, and that the sterile bag with the reaction medium inside is treated within a fluid exposed to the ultrasonic field.

7. Application of a method as defined in on or more of the claims 1 to 6 for producing lipid versicles for therapeutial purposes loaded with an effective substance such as inositolhexaphosphate and capable of fusing with red blood cells.

8. An apparatus for carrying-out the method as defined in claim 1, comprising a treatment container including the reaction fluid and at least one electric-acustic, by an electric generator supplied transmitter for producing an ultrasonic field in the reaction medium, characterized in that as a sound receiver an acustic-electric transducer (18; 37) is arranged within the treatment container (3; 30), the output signal of said transducer being used for controlling the ultrasonic intensity and frequency in the reaction fluid.

9. An apparatus as defined in claim 8, characterized by an control-amplifier (21; 39) including nominal value — actuel value comparator, the amplifier (21; 39) being connected with the electric generator (23; 43).

10. An apparatur as defined in claim 8 and 9, characterized in that the acustic-electric transducer (18; 37) is composed of a piezoelectric disk arranged at the end of a tube so that its most sound-sensitive direction faces against the sound generating means.

11. Apparatus as defined in claim 8 to 10, characterized in that the ultrasound generating means and the treatment container (3; 30) including the medium have geometries which are selected so as to avoid a reflection-caused formation of ultrasonic waves in the medium with a frequency deviating from the nominal frequency and/or with a phase which is offset relative to the phase of the primary ultrasonic waves in the medium.

12. Apparatus as defined in claim 8 to 11, characterized in that the treatment container (3; 30) has double walls with hollow spaces therein through which runs a cooling medium.

13. Apparatus as defined in claim 8 to 12, characterized in that the treatment container (3) is closed from the outer atmosphere and comprises inlet means (13) for supplying an inert gas into the gas-filled space above the reaction medium.

**Revendications**

1. Procédé pour préparer de vésicules lipidiques à partir de membranes biologiques ou de suspensions lipidiques, dans lequel les suspensions ou particules lipidiques à désintégrer sont soumises à un traitement aux ultra-sons dans un liquid de dispersion à l'intérieure d'un récipient à une températur essentiellement constante, caractérisé en ce qu'on détermine, dans le liquide de dispersion, la fréquence et l'intensité optimales d'ultra-son pour obtenir la dimension respectivement la repartition des dimensions des vésicules effectives pour le but souhaité, et qu'on maintient constantes la fréquence et l'intensité d'ultra-son optimales ainsi trouvées, les autres conditions de traitement étant les mêmes, en mesurant continuellement la valeur effective de la fréquence et de l'intensité du champ ultrasonic dans le milieu de réaction, et en réglant la puissance de sortie et la fréquence du générateur électrique alimentant le generateur des ultra-sons en dépendance de ladite valeur effective.

2. Procédé selon revendication 1, caractérisé en ce qu'on règle générateur alimentant le générateur d'ultra-sons à l'aide d'un circuit de réglage automatique à comparaison valeur de consigne — valeur effective.

3. Procédé selon revendication 1 ou 2, caractérisé en ce qu'on utilisé un capteur piézoélectrique de son plongé dans le liquide de réaction, pour mesurer l'intensité et la fréquence des ultra-sons.

4. Procédé selon revendication 3, caractérisé en ce que le capteur piézoélectrique de son est placé au sol du récipient de réaction de telle manière que la direction de sa sensibilite la plus élevée est dirigée vers le générateur des ultra-son, ce dernier étant plongé dans le tiers supérieur du milieu.

5. Procédé selon une ou plusieures des revendications 1 à 4, caractérisé en ce que le milieu de réaction est traité dans une atmosphére de gaz inerte.

6. Procédé selon une ou plusieures des revendications 1 à 5, caractérisé en ce que le milieu de réaction est sterilisé et enfermé dans un sac stérile, et que le sac stérile contenant le milieu de réaction est traité à l'intérieure d'un liquide exposé au champ des ultra-sons.

7. Application du procédé selon une ou plusieures des revendications 1 à 6 pour préparer des vésicules lipidiques déstinées aux buts thérapeutiques, chargées d'un médicament comme inositolhexaphosphat et susceptibles à la fusion avec de globules rouges du sang.

8. Dispositif pour la mise en oeuvre du procédé selon revendication 1, comprenant un récipient pour le liquide de réaction et au moins un transducteur électroacoustique alimenté par un générateur électrique, pour créer un champ des ultra-sons dans le milieu de réaction, caractérisé en ce qu'un capteur acoustique-électrique (18; 37) est disposé à l'intérieure du récipient de réaction (3; 30), le signal de sortie dudit capteur (18; 37) servant de régler l'intensité et la fréquence des ultra-sons dans le liquide de réaction.

9. Dispositif selon revendication 8, caractérisé par un amplificateur de réglage (21; 39) à comparaison valeur de consigne — valeur effective, l'amplificateur de réglage étant lié au générateur électrique (23; 43).

10. Dispositif selon revendication 8 et 9, caractérisé en ce que le capteur acoustique-électrique (18; 37) consiste en une piézo-disque disposée à l'extrémité d'un tube, la direction la plus sensible à la pression des sons étant dirigée vers le générateur des vibrations.

11. Dispositif selon revendication 8 à 10, caractérisé en ce que la géometrie du générateur des ultra-sons ainsi que la géométrie du récipient de traitement (3; 30) contenant le milieu sont choisies de telle sorte que la formation, à la suite de réflexions, d'ondes ultrasoniques ayant une fréquence différente de la fréquence de consigne et/ou une phase déviee vis-à-vis de la phase des ondes ultrasoniques primaires, soit évitée dans le milieu exposé aux ultra-sons.

12. Dispositif selon revendication 8 à 11, caractérisé en ce que le récipient de traitement (3; 30) présente des double-parois traversés par un liquide de refroidissement.

13. Dispositif selon revendication 8 à 12, caractérisé en ce que le récipient de traitement (3) est fermé vis-à-vis de l'atmosphére extérieure et présente une conduite (13) déstinée à l'alimentation de gaz inerte à l'épace de gaz au dessus du milieu de réaction (1) exposé aux ultra-sons.

*Fig. 1*

# Fig. 2